# FASCICULE DE BREVET EUROPEEN

(11) **EP 1 457 231 B1**
(45) Date de publication et mention de la délivrance du brevet: **13.05.2009**
(21) Numéro de dépôt: 04290690.9
(22) Date de dépôt: 12.03.2004
(51) Int. Cl.: A61M 27/00

(54) **Valve implantable pour le traitement de l'hydrocéphalie**
Implantierbares Ventil zur Behandlung von Hydrocephalus
Implantable valve for the treatment of hydrocephalus

(30) Priorité: 12.03.2003 FR 0303057
(43) Date de publication de la demande: 15.09.2004
(73) Titulaire: Integra Neurosciences Implants (France) SA, 06410 Biot (FR)
(72) Inventeur: Lecuyer, Alain, 06130 Grasse (FR)
(74) Mandataire: Geismar, Thierry

(56) Documents cités:
- EP-A- 1 092 450
- FR-A- 2 746 659
- US-A- 4 551 128
- US-A- 4 772 257
- US-A- 6 050 969
- US-B2- 6 485 449

## Description

La présente invention concerne une valve implantable dans le corps d'un patient pour le traitement de l'hydrocéphalie, et plus particulièrement une telle valve du type comprenant :
- un boîtier formant une cavité ;
- une membrane de séparation montée à sa périphérie sur le boîtier, pourvue d'un orifice et délimitant dans ladite cavité une chambre amont et une chambre aval ;
- ledit boîtier formant dans la chambre amont un siège pour la membrane, ledit siège entourant ledit orifice ;
- une tige de section variable agencée pour pénétrer axialement dans ledit orifice ;
- des moyens de support de la tige.

On connaît déjà de telles valves, notamment par le document FR-A-2 746 659. Les valves de ce type possèdent de façon connue une caractéristique de fonctionnement pression différentielle/débit en plusieurs zones.

Lorsque la pression différentielle entre la chambre amont et la chambre aval croît à partir de zéro, dans un premier temps aucun écoulement ne se produit tant que cette pression différentielle est insuffisante pour décoller la membrane de son siège.

Puis, à partir d'un certain seuil de pression différentielle, le profil de la tige est tel que l'on commence à avoir une zone dans laquelle la pression différentielle reste pratiquement constante pour un débit qui croît rapidement. Arrivé à un certain débit, qui est le débit de régulation de la valve, la pression augmente à débit sensiblement constant.

Enfin, au-delà d'un nouveau seuil de pression différentielle, l'extrémité libre de la tige sort de l'orifice de la membrane. Il en résulte une pression différentielle maximum, pratiquement indépendante du débit qui croît à la demande.

Dans les valves connues, la tige est supportée par un élément du boîtier. Son réglage axial, qui détermine la zone de pression à débit sensiblement constant, c'est-à-dire le débit de régulation de la valve, est effectué à la fabrication de façon définitive.

Il est par ailleurs connu des valves réglables telles que décrites dans le document US 4,551,128. Ces valves permettent de faire varier la géométrie de la membrane par un système de vis, ou de faire varier la position de la tige selon un mouvement de pivotement.

Lorsqu'il s'avère, après implantation de la valve, que son débit de régulation est mal, ou n'est plus, adapté à l'état du patient, il est nécessaire d'explanter la valve afin de pourvoir à son remplacement. Une telle opération est relativement lourde et il est donc souhaitable de pouvoir l'éviter dans la mesure du possible.

L'invention vise à pallier cet inconvénient.

Plus particulièrement, l'invention a pour but de fournir une valve du type décrit ci-dessus, qui d'une part soit réglable après implantation, et d'autre part dont le réglage puisse être réalisé de façon non invasive.

Il est connu dans l'état de la technique des moyens moteurs non-invasifs tels que décrits par les documents US 6,050,969 et US 6,485,449. Ces moyens utilisent des moteurs électromagnétiques implantés dans le corps du patient et contrôlés par un dispositif d'électro-aimants.

A cet effet, l'invention a pour objet une valve implantable dans le corps d'un patient pour le traitement de l'hydrocéphalie, du type comprenant:
un boîtier formant une cavité;
   - une membrane de séparation montée à sa périphérie sur le boîtier, pourvue d'un orifice et délimitant dans ladite cavité une chambre amont et une chambre aval
   - ledit boîtier formant dans la chambre amont un siège pour la membrane, ledit siège entourant ledit orifice;
   - une tige de section variable agencée pour pénétrer axialement dans ledit orifice;
   - des moyens de support de la tige ;
caractérisée par le fait qu'elle comprend des moyens de déplacement axial des moyens de support de la tige, des moyens moteurs pour entraîner lesdits moyens de déplacement, lesdits moyens moteurs étant agencés pour être activés depuis l'extérieur du corps du patient de manière à permettre le réglage de la valve de manière non invasive, et lesdits moyens de support de la tige comprenant une pluralité de bras de support élastiques radiaux, fixés à une première de leurs extrémités audit boîtier et supportant un manchon de support de la tige à leur autre extrémité.

On comprend qu'en déplaçant axialement la tige par rapport au plan de l'orifice ménagé dans le diaphragme, on décale par rapport à l'axe des débits la courbe caractéristique pression différentielle / débit. On règle ainsi le débit de régulation de la valve.

Par ailleurs, ce réglage est réalisé de façon non invasive, depuis l'extérieur du corps du patient. Il peut donc être effectué après implantation de la valve.

Plus particulièrement, lesdites premières extrémités des bras de support peuvent être solidaires d'un anneau de fixation adjacent au bord de ladite membrane.

Egalement dans un mode de réalisation particulier de l'invention, lesdits moyens de déplacement peuvent comprendre un levier monté sur ledit boîtier à une première de ses extrémités.

Egalement dans un mode de réalisation particulier de l'invention, lesdits moyens de déplacement comprennent un levier monté sur ledit boîtier et en appui sur lesdits moyens de support de la tige, ledit levier coopérant avec des moyens de came desdits moyens moteurs.

Plus particulièrement, ledit levier peut comprendre une lame élastique fixée audit boîtier.

Dans un mode de réalisation particulier, ledit levier est monté sur le boîtier à une de ses extrémités, coopère avec lesdits moyens de came à son autre extrémité, et est en appui sur lesdits moyens de support de la tige dans sa partie médiane.

Egalement dans un mode de réalisation particulier, la position longitudinale des moyens de came dans la direction générale du levier est réglable.

En réglant la position longitudinale des moyens de came, on peut ainsi régler le gain de la relation entre la position des moyens moteurs et la position axiale de la tige.

On peut également prévoir des moyens élastiques pour appliquer ledit levier sur lesdits moyens de came.

Dans un mode de réalisation, ces moyens élastiques comportent au moins une lame élastique.

Dans le cas où les moyens de fixation de la tige comprennent des bras élastiques solidaires d'un anneau de fixation, ladite lame élastique peut être réalisée d'une seule pièce avec ledit anneau de fixation.

Plus particulièrement, le boîtier et l'anneau de fixation peuvent comporter des saillies et des encoches complémentaires pour l'indexation angulaire de l'anneau par rapport au boîtier.

Dans un mode de réalisation particulier, lesdits moyens moteurs comprennent un moteur magnétique.

Ces moyens moteurs peuvent également comprendre au moins une came motorisée agencée pour coopérer avec un suiveur de came desdits moyens de déplacement.

Plus particulièrement, ladite came motorisée peut comprendre une platine agencée pour être entraînée en rotation par un moteur, ladite platine comportant au moins deux nervures radiales de hauteurs différentes agencées pour coopérer avec ledit suiveur de came.

Lorsque les moyens de déplacement comprennent un levier comme exposé précédemment, ladite platine peut comporter deux paires de nervures radiales sensiblement perpendiculaires, les deux nervures de chaque paire étant alignées de part et d'autre de l'axe de rotation de la platine et étant sensiblement de la même hauteur, différente de la hauteur des nervures de l'autre paire, et ledit levier former, dans sa partie où il coopère avec ladite came, deux branches coopérant chacune avec une des nervures d'une desdites paires de nervures.

Plus particulièrement, la valve peut dans ce cas comporter deux lames élastiques en appui chacune sur l'une des branches dudit levier.

On décrira maintenant, à titre d'exemple non limitatif, un mode de réalisation particulier de l'invention, en référence aux dessins schématiques annexés dans lesquels :
- la figure 1 est une vue en coupe longitudinale d'une valve selon l'invention ;
- la figure 2 est une vue à plus grande échelle du détail II de la figure 1 ;

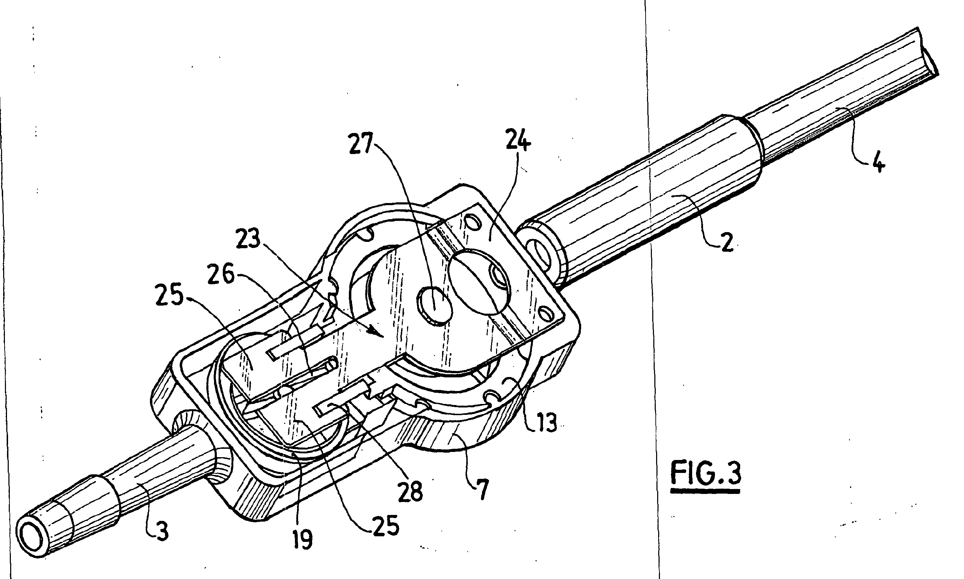

- la figure 3 est une vue en perspective de dessous du mécanisme de cette valve ;

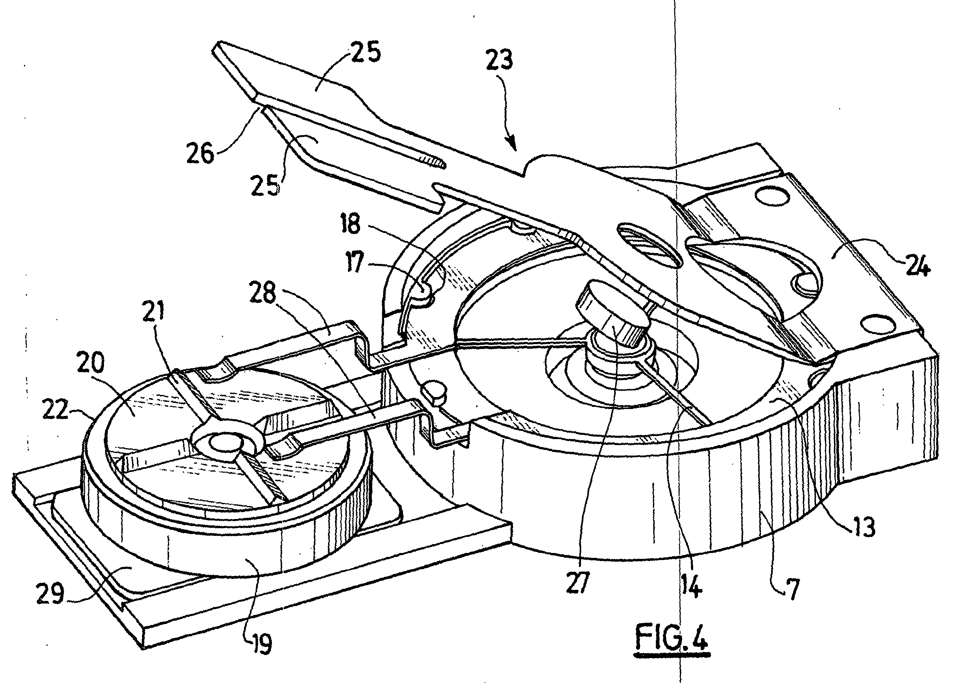

- la figure 4 est une vue en perspective de dessous de la valve ; et

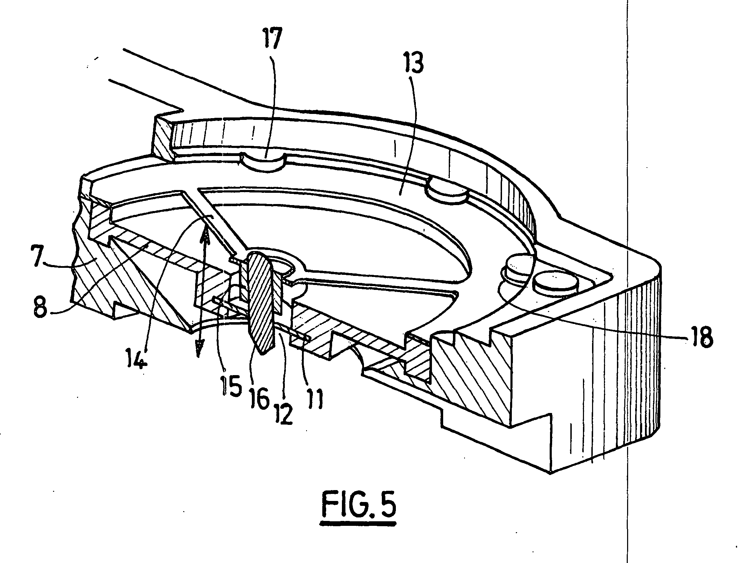

- la figure 5 est une vue partielle en coupe et en perspective du mécanisme.

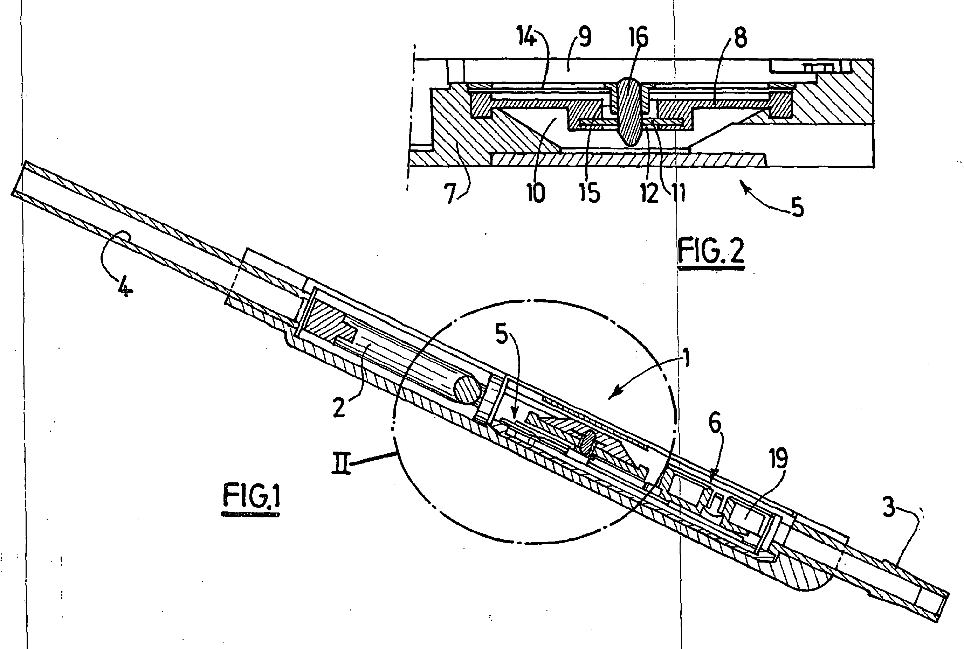

La valve 1 de la figure 1, en fait un régulateur de débit réglable, est associée ici à un clapet anti-retour 2 de type connu, et qui ne sera donc pas décrit. Le fluide pénètre dans la valve par un raccord 3, puis traverse la valve et le clapet anti-retour, et est enfin drainé par un cathéter 4.

La valve 1 est composée d'une partie 5 formant régulateur de débit, et d'une partie de réglage 6.

La partie 5 est pour l'essentiel similaire à la valve décrite dans le document FR-A-2 746 659 précité.

On voit sur la figure 2 le boîtier 7 de la valve. Une membrane élastique 8 montée à sa périphérie dans le boîtier 7 délimite dans le boîtier une chambre amont 9 et une chambre aval 10. La membrane 8 porte en son centre une rondelle 11 possédant une ouverture centrale 12. Le boîtier forme dans la chambre amont un siège de type connu, non représenté.

La bordure extérieure de la membrane 8 est retenue dans le boîtier 7 simultanément à un anneau de fixation 13 qui recouvre cette bordure. L'anneau 13 est réalisé d'une seule pièce avec trois bras de support élastiques radiaux 14 supportant à leurs extrémités intérieures un manchon de support 15 pour une tige 16 à section variable, de telle sorte que la tige 16 soit engagée dans l'ouverture 12 de la rondelle 11. La tige 16 et les bords de l'ouverture 12 déterminent donc une section de passage pour le fluide, fonction du degré d'enfoncement de la tige puisque sa section est variable.

Des saillies 17 formés dans le boîtier 7 coopèrent avec des encoches 18 complémentaires formées à la périphérie de l'anneau 13 pour indexer l'anneau 13 en rotation par rapport à l'axe de la membrane 8 et de l'anneau 13.

La valve 1 comprend par ailleurs un moteur magnétique de réglage 19 de tout type connu. Une fois la valve implantée, ce moteur permet son réglage depuis l'extérieur du corps du patient à l'aide d'un aimant convenable.

L'axe de sortie du moteur 19 supporte une platine 20 en forme de disque. La platine 20 porte deux paires de nervures radiales 21 et 22 orientées à 90° les unes des autres. Les deux nervures 21, respectivement 22, sont symétriques par rapport à l'axe de la platine 20. Les nervures 22 ont une hauteur supérieure à celle des nervures 21 au-dessus du plan de la platine 20.

Un levier 23 a une de ses extrémités 24 fixée au boîtier 7 du côté de la partie 5 de la valve 1 opposée au moteur magnétique 19. L'autre extrémité du levier 23 comporte deux branches 25 en forme de palettes, les deux branches étant séparées longitudinalement par une fente 26 d'une largeur légèrement supérieure à celle des nervures 21 et 22.

Dans sa partie centrale, le levier 23 coopère avec un organe de guidage et d'appui 27 pour déterminer l'enfoncement de la tige 16 dans la rondelle 11.

Deux pattes élastiques 28 d'une seule pièce avec l'anneau 13 font saillie du boîtier 7, passent au-dessus du levier 23, et viennent en appui sur chacune des branches 25 du levier pour les appliquer sur deux des nervures 21 ou 22 selon la position angulaire de la platine 20 déterminée par le moteur magnétique 19. Les autres nervures s'engagent dans la fente 26 du levier 23.

Enfin, le moteur magnétique 19 est disposé sur une plaque de support 29 montée par tout moyen convenable coulissante par rapport au boîtier 7.

Selon que le moteur magnétique 19 oriente les nervures 21 ou les nervures 22 perpendiculairement à la direction longitudinale du levier 23, celui-ci est en appui sur les unes ou les autres de ces nervures. Il enfonce donc plus ou moins la tige 16 dans l'orifice de la rondelle 11 et détermine ainsi, comme cela a été exposé ci-dessus, le débit de régulation de la valve 1 parmi deux débits possibles.

Le réglage précis de la valeur de l'un ou l'autre de ces débits est effectué au montage de la valve par le positionnement de la tige 16 dans son manchon de support 15.

Il est encore possible de déterminer au montage de la valve l'écart entre les deux valeurs du débit de régulation, pour deux hauteurs données des nervures 21 et 22. Cette détermination est effectuée par réglage du coulissement du moteur 19 par rapport au boîtier 7 : plus le moteur 19 est rapproché du boîtier 7, plus l'écart entre ces valeurs est grand.

## Revendications

1. Valve implantable dans le corps d'un patient pour le traitement de l'hydrocéphalie, du type comprenant :
- un boîtier (7) formant une cavité ;
- une membrane de séparation (8) montée à sa périphérie sur le boîtier, pourvue d'un orifice (12) et délimitant dans ladite cavité une chambre amont (9) et une chambre aval (10) ;
- ledit boîtier formant dans la chambre amont un siège pour la membrane, ledit siège entourant ledit orifice ;
- une tige (16) de section variable agencée pour pénétrer axialement dans ledit orifice ;
- des moyens (14, 15) de support de la tige ;
**caractérisée par le fait qu'**elle comprend des moyens de déplacement axial (23) des moyens de support de la tige, des moyens moteurs (19) pour entraîner lesdits moyens de déplacement, lesdits moyens moteurs étant agencés pour être activés depuis l'extérieur du corps du patient de manière à permettre le réglage de la valve de manière non invasive, et lesdits moyens de support de la tige comprenant une pluralité de bras de support élastiques radiaux (14), fixés à une première de leurs extrémités audit boîtier et supportant un manchon de support (15) de la tige à leur autre extrémité.

2. Valve selon la revendication 1, dans laquelle lesdites premières extrémités des bras de support sont solidaires d'un anneau de fixation (13) adjacent au bord de ladite membrane.

3. Valve selon l'une quelconque des revendications 1 et 2, dans laquelle lesdits moyens de déplacement comprennent un levier (23) monté sur ledit boîtier à une première de ses extrémités.

4. Valve selon l'une quelconque des revendications 1 à 3, dans laquelle lesdits moyens de déplacement comprennent un levier (23) monté sur ledit boîtier et en appui sur lesdits moyens de support de tige, ledit levier coopérant avec des moyens de came (20-22) desdits moyens moteurs.

5. Valve selon la revendication 4, dans laquelle ledit levier comprend une lame élastique fixée audit boîtier.

6. Valve selon l'une quelconque des revendications 4 et 5, dans laquelle ledit levier est monté sur le boîtier à une de ses extrémités, coopère avec lesdits moyens de came à son autre extrémité, et est en appui sur lesdits moyens de support de la tige dans sa partie médiane.

7. Valve selon l'une quelconque des revendications 4 à 6, dans laquelle la position longitudinale des moyens de came dans la direction générale du levier est réglable.

8. Valve selon l'une quelconque des revendications 4 à 7, comprenant des moyens élastiques (28) pour appliquer ledit levier sur lesdits moyens de came.

9. Valve selon la revendication 8, dans laquelle lesdits moyens élastiques (28) comportent au moins une lame élastique.

10. Valve selon l'ensemble de la revendication 9 et de la revendication 2, dans laquelle ladite lame élastique est réalisée d'une seule pièce avec ledit anneau de fixation.

11. Valve selon la revendication 10, dans laquelle le boîtier et l'anneau de fixation comportent des saillies (17) et des encoches (18) complémentaires pour l'indexation angulaire de l'anneau par rapport au boîtier.

12. Valve selon l'une quelconque des revendications 1 à 11, dans laquelle lesdits moyens moteurs comprennent un moteur magnétique.

13. Valve selon l'une quelconque des revendications 1 à 12, dans laquelle lesdits moyens moteurs comprennent au moins une came motorisée (20-22) agencée pour coopérer avec un suiveur de came (25) desdits moyens de déplacement.

14. Valve selon la revendication 13, dans laquelle ladite came motorisée comprend une platine (20) agencée pour être entraînée en rotation par un moteur (19), ladite platine comportant au moins deux nervures radiales (21, 22) de hauteurs différentes agencées pour coopérer avec ledit suiveur de came.

15. Valve selon l'ensemble de la revendication 14 et de l'une quelconque des revendications 4 à 11, dans laquelle ladite platine comporte deux paires de nervures radiales sensiblement perpendiculaires, les deux nervures de chaque paire étant alignées de part et d'autre de l'axe de rotation de la platine et étant sensiblement de la même hauteur, différente de la hauteur des nervures de l'autre paire, et dans laquelle ledit levier forme, dans sa partie où il coopère avec ladite came, deux branches (25) coopérant chacune avec une des nervures d'une desdites paires de nervures.

16. Valve selon la revendication 14 rattachée à l'une quelconque des revendications 9 à 11, comportant deux lames élastiques en appui chacune sur l'une des branches dudit levier.

## Claims

1. A valve implantable into a patient's body for the treatment of hydrocephaly, of the type including:
- a housing (7) forming a cavity;
- a separating membrane (8) mounted on the periphery thereof, on the housing, provided with an orifice (12) and defining in said cavity an upstream chamber (9) and a downstream chamber (10);
- said housing forming in the upstream chamber a seat for the membrane, said seat surrounding said orifice;
- a rod (16) having a variable section so arranged as to axially penetrate into said orifice;
- means (14, 15) for supporting the rod;
**characterised in that** it includes means for axially moving (23) the rod supporting means, motor means (19) for driving said motion means, said motor means being so arranged as to be activated from the outside of the patient's body so as to allow the adjustment of the valve in a non invasive manner, and said rod supporting means including a plurality of radial elastic supporting arms (14) attached at a first end to said housing and supporting a sleeve (15) supporting the rod at the other end thereof.

2. A valve according to claim 1, wherein said first ends of the supporting arms are integral with a retaining ring (13) adjacent to the edge of said membrane.

3. A valve according to any one of claims 1 and 2, wherein said motion means include a lever (23) mounted on said housing, at the first one of the ends thereof.

4. A valve according to any one of claims 1 to 3, wherein said motion means include a lever (23) mounted on said housing and resting on said rod supporting means, said lever cooperating with cam means (20-22) of said motor means.

5. A valve according to claim 4, wherein said lever includes an elastic blade fixed to said housing.

6. A valve according to any one of claims 4 and 5, wherein said lever is mounted on the housing, at one of the ends thereof, cooperates with said cam means at the other end thereof and rests on the rod supporting means in the middle part thereof.

7. A valve according to any one of claims 4 to 6, wherein the longitudinal position of the cam means in the general direction of the lever can be adjusted.

8. A valve according to any one of claims 4 to 7, including elastic means (28) for applying said lever on said cam means.

9. A valve according to claim 8, wherein said elastic means (28) includes at least one elastic blade.

10. A valve according to the whole claim 9 and claim 2, wherein said elastic blade is made in one piece with said retaining ring.

11. A valve according to claim 10, wherein the housing and the retaining ring include protrusions (17) and matching notches (18) for the angular indexing of the ring with respect to the housing.

12. A valve according to any one of claims 1 to 11, wherein said motor means includes a magnetic motor.

13. A valve according to any one of claims 1 to 12, wherein said motor means include at least one motorized cam (20-22) so arranged as to cooperate with a cam follower (25) of said motion means.

14. A valve according to claim 13, wherein said motorized cam includes a plate (20) so arranged to as to be driven into rotation by a motor (19), with said plate including at least two radial ribs (21, 22) having a different height so arranged as to cooperate with said cam follower.

15. A valve according to the combination of claim 14 and any one of claims 4 to 11, wherein said plate includes two pairs of radial ribs substantially perpendicular to each other, both ribs of each pair being aligned on either side of the plate rotation axis and being substantially of the same height, which is different from the height of the ribs of the other pair and wherein said lever forms in the part thereof where it cooperates with said cam, two branches (25) cooperating, each, with one of the ribs of one of said pairs of ribs.

16. A valve according to claim 14, according to any one of claims 9 to 11, including two elastic blades resting, each, on one of the branches of said lever.

## Patentansprüche

1. In den Körper eines Patienten einsetzbares Ventil zur Behandlung des Wasserkopf, von dem Typ umfassend:
- ein einen Hohlraum bildendes Gehäuse (7);
- eine Trennmembran (8), die an ihrer Peripherie am Gehäuse angebracht ist, mit einer Öffnung (12) versehen ist und die in dem genannten Hohlraum eine obere Kammer (9) und eine untere Kammer (10) begrenzt;
- wobei das genannte Gehäuse in der oberen Kammer einen Sitz für die Membran begrenzt, wobei der genannte Sitz die genannte Öffnung umgibt:
- einen Stift (16) mit variablem Querschnitt, der angeordnet ist, um axial in die genannte Öffnung einzudringen;
- Trägermittel (14, 15) des Stiftes;
**gekennzeichnet durch** die Tatsache, dass es axiale Verschiebungsmittel (23) der Trägermittel des Stiftes, Motormittel (19) zum Antreiben der genannten Verschiebungsmittel umfasst, wobei die genannten Motormittel angeordnet sind, um von der Außenseite des Körpers des Patienten derart aktiviert zu werden, dass die Einstellung des Ventils auf nicht invasive Weise ermöglicht wird und wobei die genannten Trägermittel des Stiftes eine Vielzahl von radialen, elastischen Trägerarmen (14) umfassen, die an einem ersten ihrer Enden an dem genannten Gehäuse befestigt sind und einen Trägerstutzen (15) des Stiftes an ihrem anderen Ende tragen.

2. Ventil gemäß Anspruch 1, in dem die genannten ersten Enden der Trägerarme fest mit einem Befestigungsring (13) verbunden sind, der am Rand der genannten Membran anliegt.

3. Ventil gemäß Anspruch 1 und 2, in dem die genannten Verschiebungsmittel einen Hebel (23) umfassen, der auf dem genannten Gehäuse an einem ersten seiner Enden angebracht ist.

4. Ventil gemäß Anspruch 1 bis 3, in dem die genannten Verschiebungsmittel einen Hebel (23) umfassen, der auf dem genannten Gehäuse und aufstützend auf den genannten Trägermitteln des Stiftes angebracht ist, wobei der genannte Hebel mit den Nockenmitteln (20 - 22) der genannten Motormittel zusammenwirkt.

5. Ventil gemäß Anspruch 4, in dem der genannte Hebel ein an dem genannten Gehäuse befestigtes elastisches Blatt umfasst.

6. Ventil gemäß Anspruch 4 und 5, in dem der genannte Hebel an einem seiner Enden auf dem Gehäuse angebracht ist, mit den genannten Nockenmitteln an seinem anderen Ende zusammenwirkt und sich auf den genannten Trägermitteln des Stiftes in seinem medianen Teil aufstützt.

7. Ventil gemäß Anspruch 4 bis 6, in dem die Längsposition der Nockenmittel in der allgemeinen Richtung des Hebels einstellbar ist.

8. Ventil gemäß Anspruch 4 bis 7 mit elastischen Mitteln (28) zur Anwendung des genannten Hebels auf die genannten Nockenmittel.

9. Ventil gemäß Anspruch 8, in dem die genannten elastischen Mittel (28) wenigstens ein elastisches Blatt umfassen.

10. Ventil gemäß der Struktur des Anspruchs 9 und des Anspruchs 2, in dem das genannte elastische Blatt aus einem einzigen Stück mit dem genannten Befestigungsring realisiert wird.

11. Ventil gemäß Anspruch 10, in dem das Gehäuse und der Befestigungsring Vorsprünge (17) und komplementäre Einkerbungen (18) für die winkelförmige Indexierung des Rings im Verhältnis zum Gehäuse umfassen.

12. Ventil gemäß Anspruch 1 bis 11, in dem die genannten Motormittel einen magnetischen Motor umfassen.

13. Ventil gemäß Anspruch 1 bis 12, in dem die genannten Motormittel wenigstens eine motorisierte Nocke (20 - 22) umfassen, die angeordnet ist, um mit einem Nockennachlaufgerät (25) der genannten Verschiebungsmittel zusammenzuwirken.

14. Ventil gemäß Anspruch 13, in dem die genannte motorisierte Nocke eine Platine (20) umfasst, die angeordnet ist, um in Rotation durch einen Motor (19) angetrieben zu werden, wobei die genannte Platine wenigstens zwei radiale Rippungen (21, 22) unterschiedlicher Höhe umfasst, die angeordnet sind, um mit dem genannten Nockenlaufgerät zusammenzuwirken.

15. Ventil gemäß Anspruch 14 und Anspruch 4 bis 11, in dem die genannte Platine zwei Paare deutlich lotrechter radialer Rippungen umfasst, wobei die zwei Rippungen jedes Paares auf jeder Seite der Rotationsachse der Platine angeordnet sind und deutlich dieselbe Höhe aufweisen, die unterschiedlich von der Höhe der Rippungen des anderen Paares ist, und in dem der genannte Hebel in seinem Teil, in dem er mit der genannten Nocke zusammenwirkt, zwei Zweige (25) bildet, die jeweils mit einer der Rippungen eines der Paare der Rippungen zusammenwirken.

16. Ventil gemäß Anspruch 14, das mit einem der Ansprüche 9 bis 11 verbunden ist und zwei elastische Blätter umfasst, die sich jeweils auf einen der Zweige des genannten Hebels aufstützen.
